(19) Europäisches Patentamt
European Patent Office
Office européen des brevets

(11) EP 1 365 763 B1

(12) EUROPEAN PATENT SPECIFICATION

(45) Date of publication and mention of the grant of the patent:
**26.11.2008 Bulletin 2008/48**

(21) Application number: **02719776.3**

(22) Date of filing: **06.02.2002**

(51) Int Cl.:
***A61K 31/47*** *(2006.01)* ***A61K 31/505*** *(2006.01)*
***A61K 31/135*** *(2006.01)*

(86) International application number:
**PCT/EP2002/001248**

(87) International publication number:
**WO 2002/064142 (22.08.2002 Gazette 2002/34)**

(54) **FARNESYL PROTEIN TRANSFERASE INHIBITOR COMBINATIONS WITH ANTIESTROGEN AGENTS**

FARNESYL-PROTEIN-TRANSFERASE HEMMER IN KOMBINATION MIT ANTIÖSTROGENEN

COMBINAISONS D'INHIBITEURS DE LA FARNESYLE PROTEINE TRANSFERASE AVEC AGENTS ANTI-OESTROGENE

(84) Designated Contracting States:
**AT BE CH CY DE DK ES FI FR GB GR IE IT LI LU MC NL PT SE TR**
Designated Extension States:
**LT LV MK RO SI**

(30) Priority: **15.02.2001 US 268839 P**

(43) Date of publication of application:
**03.12.2003 Bulletin 2003/49**

(73) Proprietor: **Janssen Pharmaceutica NV**
**2340 Beerse (BE)**

(72) Inventor: **END, David, William**
**Ambler, PA 19002 (US)**

(74) Representative: **Daelemans, Frank F.R. et al**
**Janssen Pharmaceutica N.V.,**
**Patent Department,**
**Turnhoutseweg 30**
**2340 Beerse (BE)**

(56) References cited:

| | |
|---|---|
| **WO-A-00/25789** | **WO-A-00/39082** |
| **WO-A-01/56552** | **WO-A-01/62234** |
| **WO-A-97/16443** | **WO-A-97/21701** |
| **WO-A-98/40383** | **WO-A-98/49157** |
| **WO-A-99/08682** | **WO-A-99/32114** |

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 365 763 B1

Printed by Jouve, 75001 PARIS (FR)

## Description

The present invention is concerned with combinations of a farnesyl transferase inhibitor and an antiestrogen agent for inhibiting the growth of tumor cells, and useful in the treatment of cancer.

Oncogenes frequently encode protein components of signal transduction pathways which lead to stimulation of cell growth and mitogenesis. Oncogene expression in cultured cells leads to cellular transformation, characterized by the ability of cells to grow in soft agar and the growth of cells as dense foci lacking the contact inhibition exhibited by non-transformed cells. Mutation and/or overexpression of certain oncogenes is frequently associated with human cancer. A particular group of oncogenes is known as *ras* which have been identified in mammals, birds, insects, mollusks, plants, fungi and yeasts. The family of mammalian *ras* oncogenes consists of three major members ("isoforms") : H-*ras*, K-*ras* and N-*ras* oncogenes. These *ras* oncogenes code for highly related proteins generically known as p21$^{ras}$. Once attached to plasma membranes, the mutant or oncogenic forms of p21$^{ras}$ will provide a signal for the transformation and uncontrolled growth of malignant tumor cells. To acquire this transforming potential, the precursor of the p21$^{ras}$ oncoprotein must undergo an enzymatically catalyzed farnesylation of the cysteine residue located in a carboxyl-terminal tetrapeptide. Therefore, inhibitors of the enzyme that catalyzes this modification, farnesyl protein transferase, will prevent the membrane attachment of p21$^{ras}$ and block the aberrant growth of *ras*-transformed tumors. Hence, it is generally accepted in the art that farnesyl transferase inhibitors can be very useful as anticancer agents for tumors in which *ras* contributes to transformation.

Since mutated, oncogenic forms of *ras* are frequently found in many human cancers, most notably in more than 50 % of colon and pancreatic carcinomas (Kohl et al., Science, vol 260, 1834 - 1837, 1993), it has been suggested that farnesyl tranferase inhibitors can be very useful against these types of cancer. Following further investigations, it has been found that a farnesyl transferase inhibitor is capable of demonstrating antiproliferative effects *in vitro* and antitumor effects *in vivo* in a variety of human tumor cell lines with and without ras gene mutations.

WO-97/21701 describes the preparation, formulation and pharmaceutical properties of farnesyl protein transferase inhibiting (imidazoly-5-yl)methyl-2-quinolinone derivatives of formulas (I), (II) and (III), as well as intermediates of formula (II) and (III) that are metabolized *in vivo* to the compounds of formula (I). The compounds of formulas (I), (II) and (III) are represented by

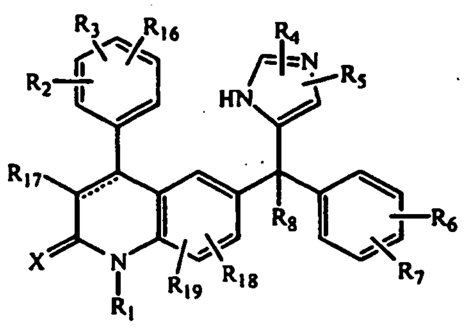

(I)

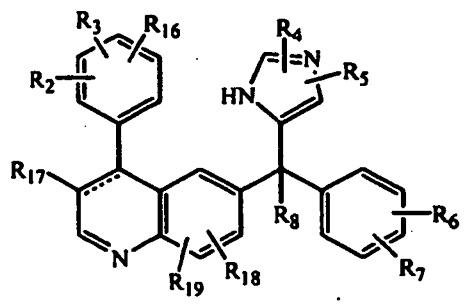

(II)

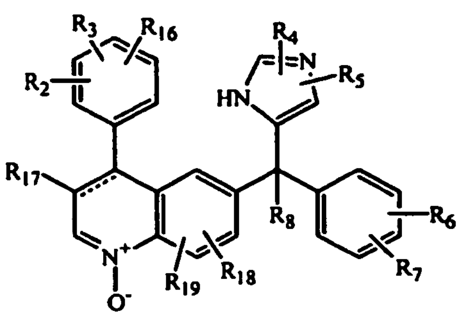

(III)

the pharmaceutically acceptable acid or base addition salts and the stereochemically isomeric forms thereof, wherein the dotted line represents an optional bond;

X is oxygen or sulfur;

$R^1$ is hydrogen, $C_{1-12}$alkyl, $Ar^1$, $Ar^2C_{1-6}$alkyl, quinolinyl$C_{1-6}$alkyl, pyridyl$C_{1-6}$alkyl, hydroxy$C_{1-6}$alkyl, $C_{1-6}$alkyloxy$C_{1-6}$alkyl, mono- or di($C_{1-6}$alkyl)amino$C_{1-6}$alkyl, amino$C_{1-6}$alkyl, or a radical of formula $-Alk^1-C(=O)-R^9$, $-Alk^1-S(O)-R^9$ or $-Alk^1-S(O)_2-R^9$, wherein $Alk^1$ is $C_{1-6}$alkanediyl,
$R^9$ is hydroxy, $C_{1-6}$alkyl, $C_{1-6}$alkyloxy, amino, $C_{1-8}$alkylamino or $C_{1-8}$alkylamino substituted with $C_{1-6}$alkyloxycarbonyl;

$R^2$, $R^3$ and $R^{16}$ each independently are hydrogen, hydroxy, halo, cyano, $C_{1-6}$alkyl, $C_{1-6}$alkyloxy, hydroxy$C_{1-6}$alkyloxy, $C_{1-6}$alkyloxy$C_{1-6}$alkyloxy, amino$C_{1-6}$alkyloxy, mono- or di($C_{1-6}$alkyl)amino$C_{1-6}$alkyloxy, $Ar^1$, $Ar^2C_{1-6}$alkyl, $Ar^2$ oxy, $Ar^2C_{1-6}$alkyloxy, hydroxycarbonyl, $C_{1-6}$alkyloxycarbonyl, trihalomethyl, trihalomethoxy, $C_{2-6}$alkenyl, 4,4-dimethyloxazolyl; or
when on adjacent positions $R^2$ and $R^3$ taken together may form a bivalent radical of formula

$-O-CH_2-O-$ (a-1),

$-O-CH_2-CH_2-O-$ (a-2),

$-O-CH=CH-$ (a-3),

$-O-CH_2-CH_2-$ (a-4),

$-O-CH_2-CH_2-CH_2-$ (a-5),

or

$-CH=CH-CH=CH-$ (a-6);

$R^4$ and $R^5$ each independently are hydrogen, halo, $Ar^1$, $C_{1-6}$alkyl, hydroxy$C_{1-6}$alkyl, $C_{1-6}$alkyloxy$C_{1-6}$alkyl, $C_{1-6}$alkyloxy, $C_{1-6}$alkylthio, amino, hydroxycarbonyl, $C_{1-6}$alkyloxycarbonyl, $C_{1-6}$alkylS(O)$C_{1-6}$alkyl or $C_{1-6}$alkyl$S(O)_2C_{1-6}$alkyl;

$R^6$ and $R^7$ each independently are hydrogen, halo, cyano, $C_{1-6}$alkyl, $C_{1-6}$alkyloxy, $Ar^2$oxy, trihalomethyl, $C_{1-6}$alkylthio, di($C_{1-6}$alkyl)amino, or
when on adjacent positions $R^6$ and $R^7$ taken together may form a bivalent radical of formula

$-O-CH_2-O-$ (c-1),

or

$-CH=CH-CH=CH-$ (c-2);

$R^8$ is hydrogen, $C_{1-6}$alkyl, cyano, hydroxycarbonyl, $C_{1-6}$alkyloxycarbonyl, $C_{1-6}$alkylcarbonyl$C_{1-6}$alkyl, cyano$C_{1-6}$alkyl, $C_{1-6}$alkyloxycarbonyl$C_{1-6}$alkyl, carboxy$C_{1-6}$alkyl, hydroxy$C_{1-6}$alkyl, amino$C_{1-6}$alkyl, mono- or di($C_{1-6}$alkyl)-amino$C_{1-6}$alkyl, imidazolyl, halo$C_{1-6}$alkyl, $C_{1-6}$alkyloxy$C_{1-6}$alkyl, aminocarbonyl$C_{1-6}$alkyl, or a radical of formula

$-O-R^{10}$ (b-1),

$-S-R^{10}$ (b-2),

$-N-R^{11}R^{12}$ (b-3),

wherein $R^{10}$ is hydrogen, $C_{1-6}$alkyl, $C_{1-6}$alkylcarbonyl, $Ar^1$, $Ar^2C_{1-6}$alkyl, $C_{1-6}$alkyloxycarbonyl$C_{1-6}$alkyl, or a radical or formula $-Alk^2-OR^{13}$ or $Alk^2-NR^{14}R^{15}$;
$R^{11}$ is hydrogen, $C_{1-12}$alkyl, $Ar^1$ or $Ar^2C_{1-6}$alkyl;
$R^{12}$ is hydrogen, $C_{1-6}$alkyl, $C_{1-16}$alkylcarbonyl, $C_{1-6}$alkyloxycarbonyl, $C_{1-6}$alkylaminocarbonyl, $Ar^1$, $Ar^2C_{1-6}$alkyl, $C_{1-6}$alkylcarbonyl$C_{1-6}$alkyl, a natural amino acid, $Ar^1$carbonyl, $Ar^2C_{1-6}$alkylcarbonyl, aminocarbonylcarbonyl, $C_{1-6}$alkyloxy$C_{1-6}$alkylcarbonyl, hydroxy, $C_{1-6}$alkyloxy, aminocarbonyl,

di($C_{1-6}$alkyl)amino$C_{1-6}$alkylcarbonyl, amino, $C_{1-6}$alkylamino, $C_{1-6}$alkylcarbonylamino, or a radical or formula -$Alk^2$-$OR^{13}$ or -$Alk^2$-$NR^{14}R^{15}$;
wherein $Alk^2$ is $C_{1-6}$alkanediyl;
$R^{13}$ is hydrogen, $C_{1-6}$alkyl, $C_{1-6}$alkylcarbonyl, hydroxy$C_{1-6}$alkyl, $Ar^1$ or $Ar^2C_{1-6}$alkyl;
$R^{14}$ is hydrogen, $C_{1-6}$alkyl, $Ar^1$ or $Ar^2C_{1-6}$alkyl;
$R^{15}$ is hydrogen, $C_{1-6}$alkyl, $C_{1-6}$alkylcarbonyl, $Ar^1$ or $Ar^2C_{1-6}$alkyl;
$R^{17}$ is hydrogen, halo, cyano, $C_{1-6}$alkyl, $C_{1-6}$alkyloxycarbonyl, $Ar^1$ ;
$R^{18}$ is hydrogen, $C_{1-6}$alkyl, $C_{1-6}$alkyloxy or halo;
$R^{19}$ is hydrogen or $C_{1-6}$alkyl;
$Ar^1$ is phenyl or phenyl substituted with $C_{1-6}$alkyl, hydroxy, amino, $C_{1-6}$alkyloxy or halo; and
$Ar^2$ is phenyl or phenyl substituted with $C_{1-6}$alkyl, hydroxy, amino, $C_{1-6}$alkyloxy or halo.

Many breast cancers have estrogen receptors and growth of these tumors can be stimulated by estrogen. Antiestrogen agents have therefore been proposed and used for the treatment of cancers especially breast cancer. One of the most widely used of such agents is tamoxifen which is a competitive inhibitor of estradiol binding to the estrogen receptor (ER). When bound to the ER, tamoxifen induces a change in the three-dimensional shape of the receptor, inhibiting its binding to the estrogen responsive element (ERE) on DNA. Under normal physiological conditions, estrogen stimulation increases tumor cell production of transforming growth cell b (TGF-b), an autocrine inhibitor of tumor cell growth. By blocking these pathways, the net effect of tamoxifen treatment is to decrease the autocrine stimulation of breast cancer growth. In addition, tamoxifen decreases the local production of insulin-like growth factor (IGF-1) by surrounding tissues: IGF-1 is a paracrine growth factor for the breast cancer cell (Jordan and Murphy, Endocr. Rev., 1990, 11; 578-610). Tamoxifen is the endocrine treatment of choice for post-menopausal women with metastatic breast cancer or at a high risk of recurrences from the disease. Tamoxifen is also used in pre-menopausal women with ER-positive tumors. There are various potential side-effects of long-term tamoxifen treatment for example the possibility of endometrial cancer and the occurrence of thrombo-embolic events. Thus, although tamoxifen has been widely used as a chemotherapeutic agent in humans, it is not therapeutically effective in all patients or against all types of tumors. Other estrogen receptor antagonists or selective estrogen receptor modulators include toremifene, droloxifene, faslodex and raloxifene.

In postmenopausal women, the principal source of circulating estrogen is from conversion of adrenal and ovarian androgens (androstenedione and testosterone) to estrogens (estrone and estradiol) by the aromatase enzyme in peripheral tissues. Estrogen deprivation through aromatase inhibition or inactivation is an effective and selective treatment for some postmenopausal patients with hormone-dependent breast cancer. Examples of aromatase inhibitors or inactivators include exemestane, anastrozole, letrozole and vorozole.

The term "antiestrogen agent" is used herein to include not only estrogen receptor antagonists and selective estrogen receptor modulators but also aromatase inhibitors as discussed above.

WO-01/45740 describes compositions and methods for treating and/or preventing breast cancer including compositions comprising at least one selective estrogen receptor modulator for example tamoxifen and at least one farnesyl transferase inhibitor for example FTI-277.

There is a need to increase the inhibitory efficacy of antiestrogen agents against tumor growth and also to provide a means for the use of lower dosages of such agents to reduce the potential of adverse toxic side effects to the patient.

It is an object of the invention to provide a therapeutic combination of an antiestrogen agent and a certain farnesyl transferase inhibitor which has an advantageous inhibitory effect against tumor cell growth, in comparison with the respective effects shown by the individual components of the combination.

According to the invention therefore we provide a combination of an antiestrogen agent and a farnesyl transferase inhibitor, namely (+)-6-[amino(4-chlorophenyl)(1-methyl-1*H*-imidazol-5-yl)methyl]-4-(3-chlorophenyl)-1-methyl-2(1*H*)-quinolinone (Compound 75 in Table 1 of the Experimental part of WO-97/21701); or a pharmaceutically acceptable acid addition salt thereof.

The above farnesyl transferase inhibitor can be converted into its pharmaceutically acceptable acid addition salts by treatment with an appropriate acid. Appropriate acids comprise, for example, inorganic acids such as hydrohalic acids, e.g. hydrochloric or hydrobromic acid; sulfuric; nitric; phosphoric and the like acids; or organic acids such as, for example, acetic, propanoic, hydroxyacetic, lactic, pyruvic, oxalic, malonic, succinic (*i.e.* butanedioic acid), maleic, fumaric, malic, tartaric, citric, methanesulfonic, ethanesulfonic, benzenesulfonic, *p*-toluenesulfonic, cyclamic, salicylic, *p*-aminosalicylic, pamoic and the like acids.

A particularly preferred antiestrogen agent for use in accordance with the invention is tamoxifen. Tamoxifen is commercially available for example from AstraZeneca plc under the trade name Nolvadex and may be prepared for example as described in GB Patent Specifications 1064629 and 1354939 or by processes analogous thereto. Other antiestrogen agents include faslodex commercially available for example from AstraZeneca plc under the trade name

Fulvestrant, raloxifene commercially available for example from Eli Lilly under the trade name Evista, toremifene commercially available for example from Schering Corporation under the trade name Fareston, and the tamoxifen analog droloxifene. Aromatase inhibitors include letrozole, anastrozole commercially available for example from AstraZeneca plc under the trade name Arimidex, exemestane commercially available for example from Pharmacia & Upjohn under the trade name under the trade name Aromasin, and vorozole.

The present invention also relates to combinations according to the invention for use in medical therapy for example for inhibiting the growth of tumor cells.

The present invention also relates to the use of combinations according to the invention for the preparation of a pharmaceutical composition for inhibiting the growth of tumor cells.

This invention further relates to the use of combinations according to the invention for inhibiting the abnormal growth of cells, including transformed cells, by administering an effective amount of a combination according to the invention. Abnormal growth of cells refers to cell growth independent of normal regulatory mechanisms (e.g. loss of contact inhibition). This includes the abnormal growth of : (1) tumor cells (tumors) expressing an activated *ras* oncogene; (2) tumor cells in which the *ras* protein is activated as a result of oncogenic mutation of another gene; (3) benign and malignant cells of other proliferative diseases in which aberrant *ras* activation occurs. Furthermore, it has been suggested in literature that *ras* oncogenes not only contribute to the growth of of tumors *in vivo* by a direct effect on tumor cell growth but also indirectly, *i.e.* by facilitating tumor-induced angiogenesis (Rak. J. et al, Cancer Research, 55, 4575-4580, 1995). Hence, pharmacologically targetting mutant *ras* oncogenes could conceivably suppress solid tumor growth *in vivo*, in part, by inhibiting tumor-induced angiogenesis.

In particular, this invention relates to the use of combinations according to the invention for inhibiting the growth of tumors expressing an activated *ras* oncogene by the administration of an effective amount of combination according to the present invention. The present invention is particularly applicable to the treatment of breast cancer including the advanced disease. Examples of other tumors which may be inhibited include, but are not limited to, lung cancer (e.g. adenocarcinoma and including non-small cell lung cancer), pancreatic cancers (e.g. pancreatic carcinoma such as, for example exocrine pancreatic carcinoma), colon cancers (e.g. colorectal carcinomas, such as, for example, colon adenocarcinoma and colon adenoma), hematopoietic tumors of lymphoid lineage (e.g. acute lymphocytic leukemia, B-cell lymphoma, Burkitt's lymphoma), myeloid leukemias (for example, acute myelogenous leukemia (AML)), thyroid follicular cancer, myelodysplastic syndrome (MDS), tumors of mesenchymal origin (e.g. fibrosarcomas and rhabdomyosarcomas), melanomas, teratocarcinomas, neuroblastomas, gliomas, benign tumor of the skin (e.g. keratoacanthomas), kidney carninoma, ovary carcinoma, bladder carcinoma and epidermal carcinoma.

This invention also relates to the use of combinations according to the invention for inhibiting proliferative diseases, both benign and malignant, wherein *ras* proteins are aberrantly activated as a result of oncogenic mutation in genes, i.e. the *ras* gene itself is not activated by mutation to an oncogenic mutation to an oncogenic form, with said inhibition being accomplished by the administration of an effective amount of a combination according to the invention, to a subject in need of such a treatment. For example, the benign proliferative disorder neurofibromatosis, or tumors in which *ras* is activated due to mutation or overexpression of tyrosine kinase oncogenes may be inhibited by the combinations according to the invention.

The antiestrogen agent and the farnesyl transferase inhibitor may be administered simultaneously (e.g. in separate or unitary compositions) or sequentially in either order. In the latter case, the two compounds will be administered within a period and in an amount and manner that is sufficient to ensure that an advantageous or synergistic effect is achieved. It will be appreciated that the preferred method and order of administration and the respective dosage amounts and regimes for each component of the combination will depend on the particular antiestrogen agent and the farnesyl transferase inhibitor being administered, the route of administration of the combination, the particular tumor being treated and the particular host being treated. The optimum method and order of administration and the dosage amounts and regime can be readily determined by those skilled in the art using conventional methods and in view of the information set out herein.

The farnesyl transferase inhibitor is advantageously administered in an effective amount of from 0.0001 mg/kg to 100 mg/kg body weight, and in particular from 0.001 mg/kg to 10 mg/kg body weight. More particularly, for an adult patient, the dosage is conveniently in the range of 50 to 500mg bid, advantageously 100 to 400 mg bid and particularly 300mg bid.

The antiestrogen agent is advantageously administered in a dosage of about 1 to 100mg daily depending on the particular agent and the condition being treated. Tamoxifen is advantageously administered orally in a dosage of 5 to 50 mg, preferably 10 to 20 mg twice a day, continuing the therapy for sufficient time to achieve and maintain a therapeutic effect. Toremifene is advantageously administered orally in a dosage of about 60mg once a day, continuing the therapy for sufficient time to achieve and maintain a therapeutic effect. Anastrozole is advantageously administered orally in a dosage of about 1 mg once a day. Droloxifene is advantageously administered orally in a dosage of about 20-100mg once a day. Raloxifene is advantageously administered orally in a dosage of about 60mg once a day. Exemestane is advantageously administered orally in a dosage of about 25mg once a day.

It is especially preferred to administer the farnesyl tranferase inhibitor at a dosage of 100 or 200mg bid for 7, 14, 21 or 28 days with a dosage of the antiestrogen agent in the ranges indicated above.

In view of their useful pharmacological properties, the components of the combinations according to the invention, i.e. the antiestrogen agent and the farnesyl transferase inhibitor may be formulated into various pharmaceutical forms for administration purposes. The components may be formulated separately in individual pharmaceutical compositions or in a unitary pharmaceutical composition containing both components. The farnesyl protein transferase inhibitor can be prepared and formulated into pharmaceutical compositions by methods known in the art and in particular according to the methods described in WO-97/21701.

The present invention therefore also relates to a pharmaceutical composition comprising an antiestrogen agent and a farnesyl tranferase inhibitor, namely(+)-6-[amino(4-chlorophenyl)(1-methyl-1*H*-imidazol-5-yl)methyl]-4-(3-chlorophenyl)-1-methyl-2(1*H*)-quinolinone together with one or more pharmaceutical carriers. To prepare pharmaceutical compositions for use in accordance with the invention, an effective amount of a particular compound, as the active ingredient is combined in intimate admixture with a pharmaceutically acceptable carrier, which carrier may take a wide variety of forms depending on the form of preparation desired for administration. These pharmaceutical compositions are desirably in unitary dosage form suitable, preferably, for administration orally, rectally, percutaneously, or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, to aid solubility for example, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wetting agent, optionally combined with suitable additives of any nature in minor proportions, which additives do not cause a significant deleterious effect to the skin. Said additives may facilitate the administration to the skin and/or may be helpful for preparing the desired compositions. These compositions may be administered in various ways, e.g., as a transdermal patch, as a spot-on, as an ointment.

It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

It may be appropriate to administer the required dose of each component of the combination as two, three, four or more sub-doses at appropriate intervals throughout the course of treatment. The sub-doses may be formulated as unit dosage forms, for example, in each case containing independently 0.01 to 500 mg, for example 0.1 to 200 mg and in particular 1 to 100mg of each active ingredient per unit dosage form.

## Anti-tumor Activity of a Combination of a Farnesyl Transferase Inhibitor and an Anti-estrogen Agent

A combination of a farnesyl transferase inhibitor, namely the compound identified as Compound 75 above (R115777), and an anti-estrogen agent, namely tamoxifen (TMX), was tested for anti-tumor activity in comparison with the activity of the individual components of the combination, as described below.

### Mice/Husbandry

Female Nude-Homo NCRNU non-ovarectimized mice 8 weeks of age were fed ad libitum water and an irradiated standard rodent diet. Mice were housed in stable microisolators on a 12 hour light cycle at 21-22° C and 40-60 % humidity.

### Tumors

Mice were inoculated subcutaneously with $1\times10^7$ MCF7 human breast carcinoma cells in the flank. Tumors were monitored initially twice a week and then daily as neoplasms reached the desired size, approximately 100mg. When the carcinomas reached a size between 62-144 mg in calculated tumor weight, the animals were pair matched into the various treatment groups (group mean tumor weights ranged from 83-85mg).

Estimated tumor weight was calculated using the formula:

$$\text{Tumor Weight (mg)} = \frac{w^2 \times l}{2}$$

w= width and 1=length in mm of a MCF7 tumor

Estrogen pellets (0.36 mg: α-estradiol, 60 day release) were implanted s.c. in the dorsal region of each mouse two days prior to MCF7 cell inoculation. Fresh estrogen pellets were implanted 64 days after the original implant. On Day 1 the estrogen pellets were removed from the two groups administered Tamoxifen (Groups 3 and 5). The pellets were left in place in Groups 1, 2 and 4, and new estrogen pellets were implanted in mice in Groups 1,2 and 4 on Day 62 of the experiment. The old pellets were not removed from these groups at the time of replacement. The MCF7 breast tumor xenograft requires exogenous estrogen to be supplied to host mice to support the progressive growth of this carcinoma.

Drugs

The vehicle was 20% beta-cyclodextrin in 0.1N HCl. Beta-cyclodextrin was added slowly to a constantly stirred approximate volume of 0.1 N HCl to yield a 40% beta-cyclodextrin solution. The mixture was covered with foil and stirred until completely dissolved (several hours). The solution was then brought to final volume and filtered (0.2μm).
R115777 was dissolved in batches sufficient for seven days dosing at a time. R115777 was pulse sonicated for 10 minutes at 4°C, filtered (0.2 μm) and transferred to sterile 15 or 50ml vials. This solution was further diluted using 20% beta-dextrin in 0.1 N HCl for lower concentration dose groups. Vials were wrapped in foil and stored at 4°C. The dosing volume (0.2ml/20g mouse) was weight adjusted.

Tamoxifen was reconstituted in corn oil at 10mg/ml. Dosing was not body weight adjusted; each mouse received 100 μL of the solution (1mg/mouse).

Treatment Plan

MCF-bearing nude mice were pair-matched on Day1 into five groups of twelve animals each. Tamoxifen was given s.c. at a dose of 1mg/mouse qd to end. R115777 was administered orally at 100mg/kg qd to end. The combination therapy group used the same regimens as were employed in the Tamoxifen and R115777 monotherapy groups. A growth control (no treatment group) and a vehicle control group were included in the study. Estrogen pellets were removed from the Tamoxifen monotherapy and the combination therapy groups on Day1, to avoid antagonizing the Tamoxifen antiestrogen effect

End point

The tumor growth inhibition (TGI) endpoint was used in this study to assess the efficacy of the various treatments. The tumor burden endpoint was set at 1.0g as measured by calliper. TGI values were determined on the last day of the study (Day 5), when all mice were under test, except those that had expired from treatment-related or procedural causes. The mice were euthanized at termination, their MCF7 tumors were excised and weighed, and the TGI values were calculated from the final group mean carcinoma actual weights (excluding those that underwent tumor shrinkage; CRs or PRs).
At excision, tumors smaller than their size on Day 1 were called PRs (partial regressions), and a mouse with no visible carcinoma was termed a CR (complete regression).

Animals recorded as CRs or PRs were not included in the TGI calculations. The following formula was used to calculate the TGI values:

$$\%TGI = \left[1 - \left(\frac{\text{Mean Net Tumor Weight}_{\text{Treated}}}{\text{Mean Net Tumor Weight}_{\text{Control}}}\right)\right] \times 100\%$$

Sample Collection

At endpoint, tumors were removed and weighed. At their endpoint, after Day 27, each tumor was cut in half with a scalpel and half was placed in fifteen to twenty volumes of 10% neutral buffered formalin. The other half was snap-frozen in liquid nitrogen and stored at -80°C. At their endpoint, after Day 30, blood was collected from the remaining

mice of Groups 3, 4 and 5 by cardiac puncture under $CO_2$ anesthesia. Serum was recovered, and stored at -80°C until the end of the study.

Results

**SUMMARY OF REGRESSION OBSERVED IN ESTABLISHED MCF-7 HUMAN BREAST TUMOR XENOGRAFTS**

| **Treatment:** | TMX 1 mg/kg | R115777 100 mg/kg | TMX + R115777 |
|---|---|---|---|
| **Regression in Individual Animals*** | 10% | 93% | 94% |
| | 78% | 100% | 94% |
| | 94% | | 78% |
| | 44% | | 95% |
| | 59% | | 88% |
| | 31% | | 31% |
| | | | 100% |

* Values indicate the magnitude of tumor regression expressed as % reduction from pretreatment tumor size Untreated control tumors showed no incidence of regression.

These data show that the tested combination unexpectedly increases cytotoxic tumor regression in comparison to the cytostatic effect of the individual components of the combination.

**TABLE 1**

| **Protocol Design For The MCF7 Study** | | | | | | | | | |
|---|---|---|---|---|---|---|---|---|---|
| Group | n | Treatment Regimen 1 | | | | Treatment Regimen 2 | | | |
| | | Agent | mg/kg | Route | Schedule | Agent | mg/kg | Route | Schedule |
| 1 | 12 | Growth Control | | | | | | | |
| 2 | 12 | Vehicle | | po | QD to end | | | | |
| 3 | 12 | Tamoxifen | 1 mg/mouse | sc | Qod to end | | | | |
| 4 | 12 | R115777 | 100 | po | QD to end | | | | |
| 5 | 12 | Tamoxifen | 1 mg/mouse | sc | Qod to end | R115777 | 100 | po | Qd to end |

## Claims

1. A combination of an antiestrogen agent and a farnesyl transferase inhibitor, namely (+)-6-[amino(4-chlorophenyl)(1-methyl-1*H*-imidazol-5-yl)methyl]-4-(3-chlorophenyl)-1-methyl-2(1*H*)-quinolinone; or a pharmaceutically acceptable acid addition salt thereof.

2. A combination as claimed in claim 1 in which the antiestrogen agent is an estrogen receptor antagonist or a selective estrogen receptor modulator.

3. A combination as claimed in claim 2 in which the antiestrogen agent is tamoxifen.

4. A combination as claimed in claim 2 in which the antiestrogen agent is faslodex, raloxifene, toremifene or droloxifene.

5. A combination as claimed in claim 1 in which the antiestrogen agent is an aromatase inhibitor

6. A combination as claimed in claim 5 in which the antiestrogen agent is letrozole, anastrozole, exemestane or vorozole.

7. A combination as claimed in any of the preceding claims in the form of a pharmaceutical composition comprising an antiestrogen agent and the said farnesyl transferase inhibitor together with one or more pharmaceutical carriers.

8. A combination as claimed in any of the preceding claims for use in medical therapy.

9. A combination as claimed in claim 8 for inhibiting the growth of tumor cells.

10. Use of a combination as claimed in any of claims 1 to 7 in the manufacture of a pharmaceutical composition for inhibiting the growth of tumor cells.

**Patentansprüche**

1. Kombination eines antiöstrogenen Mittels und eines Farnesyltransferaseinhibitors, nämlich (+)-6-[Amino-(4-chlorphenyl)(1-methyl-1*H*-imidazol-5-yl)methyl]-4-(3-chlorophenyl)-1-methyl-2(1*H*)-chinolinon, oder eines pharmazeutisch unbedenklichen Säureadditionssalzes davon.

2. Kombination nach Anspruch 1, wobei es sich bei dem antiöstrogenen Mittel um einen Östrogenrezeptorantagonisten oder einen selektiven Östrogenrezeptormodulator handelt.

3. Kombination nach Anspruch 2, wobei es sich bei dem antiöstrogenen Mittel um Tamoxifen handelt.

4. Kombination nach Anspruch 2, wobei es sich bei dem antiöstrogenen Mittel um Faslodex, Raloxifen, Toremifen oder Droloxifen handelt.

5. Kombination nach Anspruch 1, wobei es sich bei dem antiöstrogenen Mittel um einen Aromataseinhibitor handelt.

6. Kombination nach Anspruch 5, wobei es sich bei dem antiöstrogenen Mittel um Letrozol, Anastrozol, Exemestan oder Vorozol handelt.

7. Kombination nach einem der vorhergehenden Ansprüche in Form einer pharmazeutischen Zusammensetzung, die ein antiöstrogenes Mittel und den Farnesyltransferaseinhibitor zusammen mit einem oder mehreren pharmazeutischen Trägern enthält.

8. Kombination nach einem der vorhergehenden Ansprüche zur Verwendung in der medizinischen Therapie.

9. Kombination nach Anspruch 8 zur Inhibierung des Wachstums von Tumorzellen.

10. Verwendung einer Kombination nach einem der Ansprüche 1 bis 7 bei der Herstellung einer pharmazeutischen Zusammensetzung zur Inhibierung des Wachstums von Tumorzellen.

**Revendications**

1. Combinaison d'un agent anti-oestrogène et d'un inhibiteur de la farnésyl transférase, à savoir la (+)-6-[amino(4-chlorophényl)(1-méthyl-1*H*-imidazol-5-yl)méthyl]-4-(3-chlorophényl)-1-méthyl-2(1*H*)-quinoléinone, ou un sel d'addition d'acide pharmaceutiquement acceptable de celle-ci.

2. Combinaison selon la revendication 1, **caractérisée en ce que** l'agent anti-oestrogène est un antagoniste du récepteur oestrogène ou un modulateur sélectif du récepteur oestrogène.

3. Combinaison selon la revendication 2, **caractérisée en ce que** l'agent anti-oestrogène est le tamoxifène.

4. Combinaison selon la revendication 2, **caractérisée en ce que** l'agent anti-oestrogène est le faslodex, le raloxifène, le torémifène ou le droloxifène.

5. Combinaison selon la revendication 1, **caractérisée en ce que** l'agent anti-oestrogène est un inhibiteur de l'aromatase.

6. Combinaison selon la revendication 5, **caractérisée en ce que** l'agent anti-oestrogène est le létrozole, l'anastrozole, l'exémestane ou le vorozole.

7. Combinaison selon l'une quelconque des revendications précédentes, sous forme d'une composition pharmaceutique comprenant un agent anti-oestrogène et ledit inhibiteur de la farnésyl transférase, conjointement avec un ou plusieurs supports pharmaceutiques.

8. Combinaison selon l'une quelconque des revendications précédentes, destinée à être utilisée en thérapie médicale.

9. Combinaison selon la revendication 8, destinée à inhiber la croissance des cellules tumorales.

10. Utilisation d'une combinaison selon l'une quelconque des revendications 1 à 7, dans la fabrication d'une composition pharmaceutique destinée à inhiber la croissance de cellules tumorales.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9721701 A
- WO 0145740 A
- GB 1064629 A
- GB 1354939 A


### Non-patent literature cited in the description


- **KOHL et al.** *Science,* 1993, vol. 260, 1834-1837
- **JORDAN ; MURPHY.** *Endocr. Rev.,* 1990, vol. 11, 578-610
- **RAK. J. et al.** *Cancer Research,* 1995, vol. 55, 4575-4580